# EUROPEAN PATENT SPECIFICATION

(11) **EP 2 956 511 B1**
(45) Date of publication and mention of the grant of the patent: **25.04.2018**
(21) Application number: 14704276.6
(22) Date of filing: 03.02.2014
(51) Int. Cl.: C09D 5/00

(54) **HYBRID NANOCOMPOSITE MATERIALS OF AMYLOID FIBRILS AND GOLD**
HYBRID-NANOKOMPOSITMATERIALIEN AUS AMYLOID-FIBRILLEN UND GOLD, UND HERSTELLUNG DAVON
MATÉRIAUX NANOCOMPOSITES HYBRIDES DE FIBRILLES AMYLOÏDES ET D'OR ET LEUR PROCÉDÉ DE FABRICATION

(30) Priority: 12.02.2013 EP 13000719
(43) Date of publication of application: 23.12.2015
(73) Proprietor: ETH Zürich, 8092 Zürich (CH)
(72) Inventor: LI, Chaoxu, 8092 Zürich (CH); BOLISETTY, Sreenath, CH-8051 Zürich (CH); MEZZENGA, Raffaele, CH-8604 Volketswil (CH)
(74) Representative: Grimm, Siegfried
(86) International application number: PCT/CH2014/000014
(87) International publication number: WO 2014/124546

(56) References cited:
- WO-A2-00/17328
- WO-A2-02/28507
- SREENATH BOLISETTY ET AL: "Amyloid-mediated synthesis of giant, fluorescent, gold single crystals and their hybrid sandwiched composites driven by liquid crystalline interactions", JOURNAL OF COLLOID AND INTERFACE SCIENCE, vol. 361, no. 1, 17 May 2011 (2011-05-17), pages 90-96, XP055066466, ISSN: 0021-9797, DOI: 10.1016/j.jcis.2011.05.018 cited in the application
- SCHEIBEL T ET AL: "CONDUCTING NANOWIRES BUILT BY CONTROLLED SELF-ASSEMBLY OF AMYLOID FIBERS AND SELECTIVE METAL DEPOSITION", PROCEEDINGS OF THE NATIONAL ACADEMY OF SCIENCES, NATIONAL ACADEMY OF SCIENCES, US, vol. 100, no. 8, 15 April 2003 (2003-04-15), pages 4527-4532, XP001180754, ISSN: 0027-8424, DOI: 10.1073/PNAS.0431081100
- BASTUS ET AL: "Gold nanoparticles for selective and remote heating of beta-amyloid protein aggregates", MATERIALS SCIENCE AND ENGINEERING C, ELSEVIER SCIENCE S.A, CH, vol. 27, no. 5-8, 8 August 2007 (2007-08-08), pages 1236-1240, XP022190479, ISSN: 0928-4931, DOI: 10.1016/J.MSEC.2006.08.003
- IVONNE OLMEDO ET AL: "How Changes in the Sequence of the Peptide CLPFFD-NH 2 Can Modify the Conjugation and Stability of Gold Nanoparticles and Their Affinity for [beta]-Amyloid Fibrils", BIOCONJUGATE CHEMISTRY, vol. 19, no. 6, 30 May 2008 (2008-05-30), pages 1154-1163, XP055048828, ISSN: 1043-1802, DOI: 10.1021/bc800016y
- DAEKYUN LEE ET AL: "Photoconductivity of Pea-Pod-Type Chains of Gold Nanoparticles Encapsulated within Dielectric Amyloid Protein Nanofibrils of [alpha]-Synuclein", ANGEWANDTE CHEMIE INTERNATIONAL EDITION, vol. 50, no. 6, 7 February 2011 (2011-02-07), pages 1332-1337, XP055012130, ISSN: 1433-7851, DOI: 10.1002/anie.201004301

## Description

The present invention describes hybrid nanocomposite materials containing amyloid fibrils and elemental gold; and relates to environmentally friendly ("green") methods for manufacturing such materials.

It is known that gold has many industrial and commercial applications. First, Gold stimulates an ever-lasting craze not only in jewelry and decoration markets. Second, due to its combination of exceptional physical and chemical stability, unusual optical properties, catalytic and conductive properties, gold also attracts great interests in many different fields of science and technology. For example, nanoscale gold objects (such as particles, rods and platelets can serve a wide range of applications in catalysts, sensors and optoelectronic devices.

CN20111146409 discloses a method for preparing polypyrrole/gold nano composites. The nanocomposites obtained by this method have inferior properties when compared to the inventive nanocomposites; also the manufacturing is considered disadvantageous, due to the need of polypryrroles and surfactants and it is not considered "green".

CN20101274005 discloses a modification method for gold nanorods, which is characterized in that the gold nanorods are contacted with a DNA solution or a protein antigen solution, the surface of the gold nanorods carries a positive charge. The invention also relates to a gold nanorods-functional molecule composite obtained by the modification method. The composites obtained by this method have inferior properties when compared to the inventive nanocomposites; also the manufacturing is considered disadvantageous as very specific starting materials are used, unsuitable for most commercial applications. Bolisetty et al (Journal of Colloid and Interface Science2011,90-96, describe self-assembled protein-gold composites in solution. The document discloses the single crystal structure of gold, but does not teach or suggest how to obtain self-supporting or solid materials.

A number of authors describe composite materials comprising amyloid fibrils and elemental gold: Scheibel et al. (PNAS, 2003 4527-4532); Bamdad (WO02/28507); Bastus et al. (Material Science and Engineering, 2007, 1236-1240); Olmedo et al. (Bioconjugate Chem, 2008, 1154-1163); Lee et al. (Angew. Chem. Int. Ed., 2011, 1332-1337); Dobson et al (WO00/17328). All these authors fail in providing gold in the form of single crystals; making it unsuitable - or at least less suitable - for the applications described in the present invention.

Hence, it is the object of the present invention to provide improved hybrid nanocomposite materials and to provide improved methods for manufacturing such materials which also allow targeting new applications.

These objectives are achieved by the composite material as defined in claim 1 and the manufacturing method as defined in claim 6. Further aspects of the invention are disclosed in the specification and independent claims, preferred embodiments are disclosed in the specification and the dependent claims.

The present invention will be described in more detail below. It is understood that the various embodiments, preferences and ranges as provided / disclosed in this specification may be combined at will. Further, depending of the specific embodiment, selected definitions, embodiments or ranges may not apply.

As used herein, the terms "a", "an,", "the" and similar terms used in the context of the present invention (especially in the context of the claims) are to be construed to cover both the singular and plural unless otherwise indicated herein or clearly contradicted by the context.

The present invention will be better understood by reference to the **figures.**

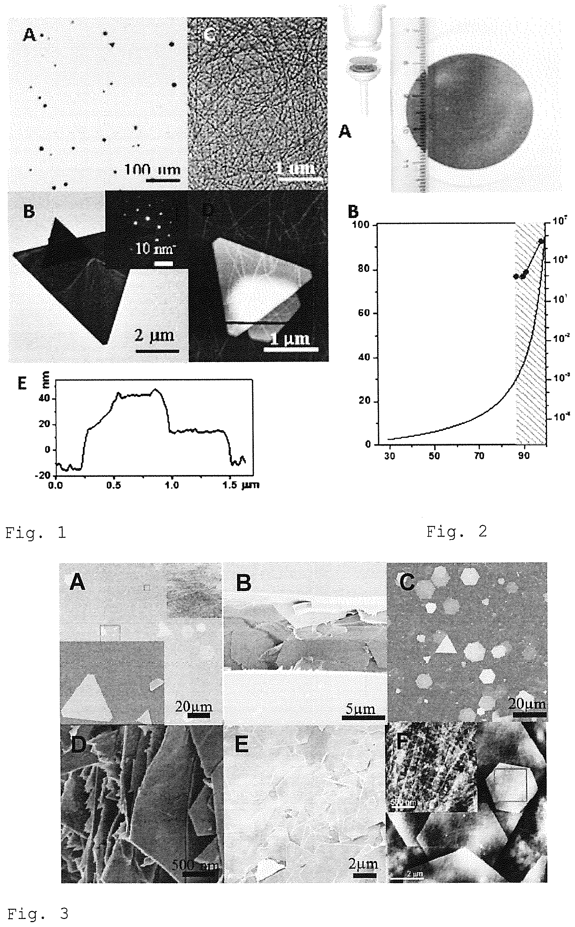

**Figure 1** shows microscopic characterization of gold nanoplatelets and amyloid fibrils in the colloidal suspension. (a) Optical microscopy image of gold single crystal nanoplatelets with high colloid stability. b) TEM image of gold single crystal nanoplatelets. The inset gives the typical single crystal diffraction pattern observed by selected area electron diffraction. (c) TEM image of amyloid fibrils in the suspension. (d) AFM image of a typical gold single crystal plate. (e) The height profile along the line in Figure Id. A plate thickness of ∼ 25 nm is indicated.
**Figure 2** shows hybrid film fabrication and corresponding electrical conductivity. (a) Filtration setup and a typical hybrid film obtained. (b) Non-linear relationship between the weight (X-axis, unit: %) and volume composition of gold platelets (Left Y-axis, unit: %) in the hybrid films and in-plane conductivity (right Y-axis, unit: S/cm) versus composition. The conductive regime is identified as the striped region; the white region identifies insulating films.
**Figure 3** shows structural characterization of hybrid films with varying gold compositions. SEM images of the hybrid films: (a) Surface and (b) fracture section with 29 wt. % gold. (c) Surface and (d) fracture section with 47 wt. % gold. (e) Surface with 87 wt. % gold. (f) AFM image of the hybrid film surface with 87 wt. % gold. The zoomed-in square regions were shown as the insets.

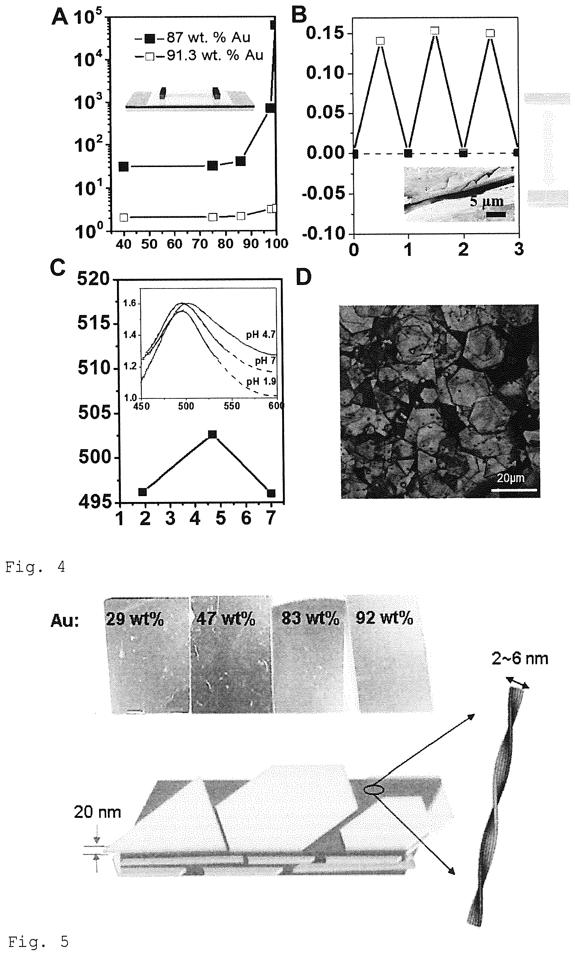

**Figure 4** shows physical properties of the hybrid films. (a) Relative humidity-dependence (x-axis, unit: %) of the resistance (y-axis, unit: **Ω**) for the films with different gold compositions. The setup is shown as the inset. (b) Humidity-controlled "ON" (open symbol) and "OFF" (solid symbol) states within three dry-wet-dry cycles (X-axis) for the film with 90 wt. % gold. Y-axis is the inverse resistance (unit: **1/Ω**). The inset SEM image gives a typical micro-scratch. (c) pH-dependence (x-axis) of the maximum of scattered surface plasmon resonance for the film with 83 wt.% gold. y-axis is the wavelength (unit: nm). The surface plasmon resonance scattering spectra are shown as the inset (scattering intensity/a.u vs. wavelength/nm). (d) Confocal optical microscopy image of the hybrid film with 87 wt. % gold. The exiting wavelength was 488 nm and the signal was collected within the wavelength range of 518-561 nm.
**Figure 5** shows schematic representation of the inventive composites, and the visual appearance thereof with a gold content ranging from 29-92 wt%.

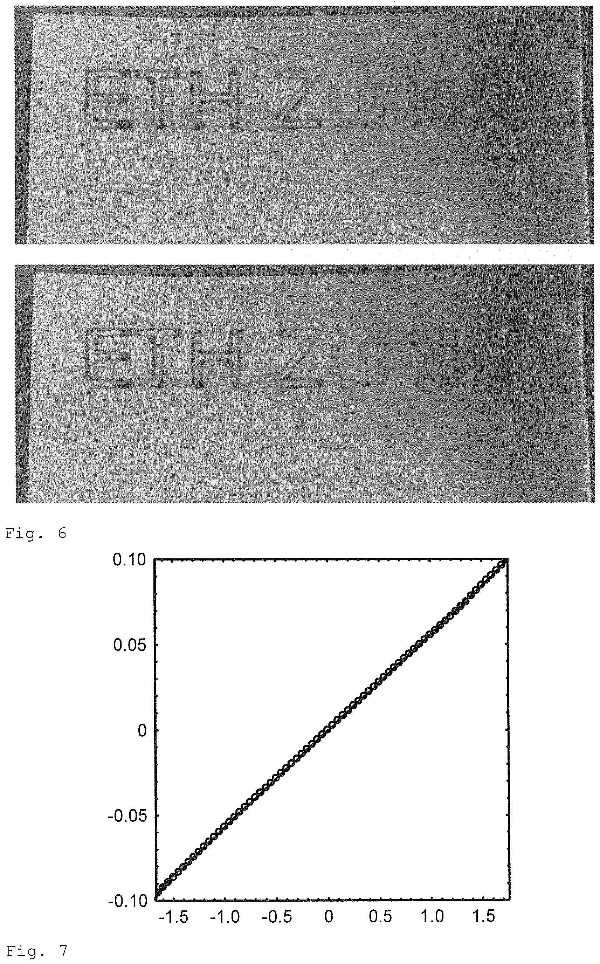

**Figure 6** shows composite materials printed with an ink jet printer as described in ex. 3. Top: letters "ETH Zurich" on paper; bottom: letters "ETH Zurich" on glass.
**Figure 7** shows the IV curve (after annealing) of a composite obtained by ink printing according to ex. 3; voltage (V) on the X-axis and current (A) on the Y-axis.

In a **first aspect,** the present invention describes hybrid nanocomposite materials ("hybrid nanocompsites", "composite materials") containing (i.e. comprising or consisting of) amyloid fibrils and elemental gold. This aspect of the invention shall be explained in further detail below:
The invention relates to biological hybrid composites made of inorganic gold platelets and protein fibrils, which are analogous to their natural counterparts selected by evolution and optimized by nature, such as bone, enamel and nacre. In particular, super large and high aspect ratio gold single crystal platelets are combined with protein amyloid fibrils to design a new class of hybrid nanocomposites. These nanocomposites are typically obtained in the form of thin films and show unprecedented properties, such as gold shining. It is further important to note that these nanocomposites are easy to filter and print, significantly facilitating its handling on a commercial scale.

**Amyloid Fibrils:** The term "amyloid fibrils" is generally known in the field and particularly describes fibrils made by proteins or peptides prevalently found in beta-sheet secondary structure. Accordingly, the term amyloid fibrils excludes native proteins.
Without being bound to theory, the roles played by amyloid fibrils are believed to be multiple: they allow reduction of gold salts into platelets, their colloidal stabilization, their tight packing and adhesion and consolidate the platelets into compact films. Advantageously, the amyloid fibrils have high aspect ratio, preferably with ≤ 10 nm in diameter and ≥ 1µm in length. Advantageously, the amyloid fibrils have a highly charged surface. The term highly charged surfaces is generally known in the field and particularly describes surfaces showing electrophoretic mobilities of the order 2 µm·cm/V·s at pH 4.

**Elemental Gold:** The inventive composites comprise gold in elemental form, i.e. oxidation state +/-0. Advantageously, the elemental gold is present in the form of gold platelets, preferably single crystal gold platelets. Such platelets have a high aspect ratio, such as 500: 1, preferably 800:1; typical sizes are up to 20 µm and the thickness only of 100 nm or less, such as 25 nm or less. Without being bound to theory, it is believed that the high aspect ratio gold single crystals provide metal conductivity, fluorescence and plasmonic features. The resulting materials have well-defined layered hierarchical structures and combine physical properties from both individual constituents, such as, for example, water-responsive and tunable conductivities from insulating to metallic levels.
The amount of elemental gold may vary over a broad range, depending on the intended use of the inventive composites. Typically, elemental gold amounts to 10-99 wt.%, preferably 30 - 99 wt.% of the total weight of the composite material. Accordingly, composite materials, of the present invention may have a gold content of 9ct, 14ct, 18ct 21ct, 21.6ct, or 22ct, for example.

**Composite Materials:** The present invention describes multi-functional, hybrid, nano-sized composites. In particular, the hybrid composites are made of 2-dimensional and 1-dimensional nanoscale building blocks, which are well organized into layered hierarchical structure. Single crystal gold nanoplatelets typically form 2D- building blocks, and amyloid fibrils typically form 1D-building blocks, see e.g. fig. 1c and 5.
The size of the nano-sized composites may vary; typically a range of 20 - 1000 nanometers is found. Without being bound to theory, it is believed this particle size contributes to its golden shiny properties, making it suitable for the applications outlined herein.
The term "hybrid composite" or "hybrid material" refers to materials comprising both, organic components as well as inorganic components in intimate contact. These hybrid composite materials may be present in various forms.
In one embodiment, the inventive composite is present as a dispersed phase in an aqueous suspension. This is typically the case during manufacturing, as outlined below.
In a further embodiment, the inventive composite is present as a self-supporting thin film. This is typically the case once manufacturing is completed. In this form, the inventive composites have an appearance and handling properties well comparable to gold foil / gold leafs.
In a further embodiment, the inventive composite is present as a coating on a substrate. A broad range of substrates may be coated, depending on the intended use of the inventive composite. The coating may be the top coating, e.g. when leaf gilding decorative articles. The coating may also be a functional layer, e.g. in a sensor or electrical device.

In a **second aspect,** the invention relates to a method of manufacturing the inventive composite materials. This shall be explained in further detail below:
The starting materials, amyloid fibrils and gold platelets are known materials. According to the inventive method, the two components were combined into a novel family of layered hybrid films via a simple vacuum-filtration process. To this end, amyloid fibrils offered several advantages over native proteins. Firstly, they allowed reducing the gold salts into colloidal nanoplatelets of very large lateral sizes (∼10¹ µm) and provided the platelets with high colloidal stability. Secondly, their exceptional adhesive properties enabled the assembly of individual platelets into homogeneous layered composites (Mostaert, A. S. et al. Nanotechnology. 2007, 18, 044010; Mostaert, A. S. et al. The Journal of Adhesion 2009, 85, 465-483). Thirdly, their remarkable stiffness and high aspect ratio, allowed them to withstand the filtration process through the vacuum membrane with only negligible losses of the organic component. By using a combination of structural and physical characterization techniques, it was confirmed that well-organized layered hierarchical structures are provided. The inventive composite materials obtained by the process show remarkable properties that none of the constituents could generate alone.

It is considered particularly advantageous that the entire manufacturing is green and eco-friendly.

It is considered particularly advantageous that the inventive method provides films of nanocomposite materials with well-organized structure and unprecedented properties.

It is further considered particularly advantageous that the obtained materials have unique optical properties, such as fluorescent and optic-grade golden colour.

Accordingly, the invention provides a process for manufacturing hybrid nanocomposite materials as described herein, comprising the steps of (a) Growing protein amyloid fibrils, preferably from β-lactoglobulin or lysozyme; (b) Growing single crystal platelets, preferably from chloroauric acid, in the presence of amyloid fibrils; (c) Optionally concentrating the thus obtained single crystal gold platelets in suspensions; and then further processing the thus obtained concentrated suspension of gold platelets-protein fibrils either by (d) filtration techniques, or (e) printing techniques or (f) casting techniques.

Accordingly, steps (a) to (c) relate to the manufacturing of the hybrid nanocomposite materials as described herein, where step (c) is optional and depends on the further processing. These hybrid nanocomposite materials may then be further processed to obtain self-supporting thin films or coatings on a substrate by method known per se, such as steps (d) or (e) or (f).

The inventive process may be accomplished by further steps, e.g. preceding step (a) or following step (d)/ (e) /(f), including purification, further processing, assembling and other process steps known to the skilled person.
**Step a:** The synthesis of amyloid fibrils is a known technology. Suitable is in particular protein hydrolysis followed by β-sheets driven fibrillation, as described e.g. in Jung et al. (Biomacromolecules. 2008, 9, 2477-2486). Suitable starting materials are food-grade proteins, which are structural stable, wide accessible and inexpensive. Such starting materials allow preparation of amyloid fibrils, such as *β*-lactoglobulin. Suitable proteins may be selected from the group consisting of *β*-lactoglobulin, lysozyme, ovalbumin, and serum albumines.
   The self-assembly process is facile and controllable. Typical process parameters include incubating protein solution (e.g. 2 wt.% *β*-lactoglobulin) for a prolonged period of time (e.g. 6 h) under acidic conditions (e.g. pH ∼ 2), low ionic strength (e.g. I ≤ 20 mM), high temperature (e.g. T ∼ 90 °C).
**Step b:** The synthesis of single crystal gold platelets is a known technology. Suitable is in particular the green chemistry method of Bolisetty et al. (Journal of Colloid and Interface Science. 2011, 361, 90-96).
   The inventors have produced single crystal gold platelets with super large size (eg. ∼10⁴ nm) and high aspect ratio (up to 10³) using a simple green method. Under controlled conditions (particularly pH, temperature, amyloid fibril concentration), amyloid fibrils can act both as a green reducing agent and a stabilization agent to synthesize the gold platelets and to provide high colloidal stability. In other terms, due to the three-fold roles played by the protein fibrils, only two materials were involved in the fabrication of these unique gold platelets as well as the inventive composites.
   Typical process parameters include mixing 0.67 wt. % amyloid fibrils of step a) and 0.066 wt.% chloroauric acid, and then incubating at pH 2 at elevated temperatures (e.g. 60 °C) for a prolonged period of time (e.g. 12 h).
**Step c:** Non-reacted starting materials may be separated, thereby concentrating the inventive composites in suspension. This may be done by simple centrifugation, and discharging / recycling the non-reacted supernantant aqueous amyloid phase. This step improves product quality.
**Step d:** The hybrid composite of the present invention was produced with the gold platelets-protein fibrils suspension via a simple vacuum-filtration process. During this process, gold platelets organize into parallel layered structures, and the remarkable stiffness and high aspect ratio of amyloid fibrils allowed them to withstand the filtration process with only negligible losses of the organic component. Their inherent adhesive properties are crucial for forming well-aligned nanoplatelet-fibril sandwiched structures. By this method, self-supporting thin films are available.
   Typical process parameters include filtrating the gold platelets-protein fibrils suspension through filtration membranes (e.g. using a vacuum pump) and drying naturally in air.
   Steps (a) to (d) are typically performed subsequently. Typically, the intermediates are not isolated. The invention thus provides for a process as described herein which is a one-pot process. This method results in a self-supporting composite material.
**Step e:** To obtain coated substrates, printing technologies may be used. Accordingly, the invention provides for a method, comprising the step of printing a suspension comprising the hybrid composites as described herein. Suitable printing techniques include ink jet printing or micro-contact printing.
   Suitable inks for this printing process include the suspensions of step (b) or step (c). Depending on the printing technique used, these suspensions may be modified by addition of further components, such as viscosity modifiers, humectants. Such ink formulations are known and may be prepared according to known methods. In a specific embodiment, the invention provides for an ink jet ink, comprising the inventive composites, water and humectant (such as PEG). Suitable amounts of components, and optional further additives, may be determined by routine experiments and depend inter alia on the printer, printer head and substrate to be printed.
   Printing technologies are particularly suited for obtaining patterned solid film motif on a substrate, such as logos, alphanumerical codes, ornaments, and the like. Accordingly, in a further embodiment, the inventive composite is part of an ink, particularly of an ink jet ink. As outlined above, the hybrid composites form stable suspensions, particularly stable aqueous suspensions. It is thus possible to provide ink formulations, where the inventive composites replace traditional pigments (such as carbon black).
**Step f:** To obtain coated substrates or self-supporting thin films, casting techniques may be used. Accordingly, the invention provides for a method, comprising the step of casting a suspension comprising the hybrid composites as described herein. Suitable casting techniques are known in the field and include slow evaporation and doctor-blading techniques.
   In a further embodiment, the invention also provides for a composite material obtainable by the method described herein, or obtained according to the method as described herein.
   In this embodiment, the amyloid fibrils are preferably prepared from food-grade proteins; preferably selected from the group consisting of *β*-lactoglobulin, lysozyme, ovalbumin, and serum albumines. It is considered particularly advantageous that broadly available, inexpensive food-grade proteins are suitable starting materials for manufacturing the inventive composites. Further, in this embodiment the single crystal gold platelets are simply prepared by reducing gold salts in an aqueous solution optionally further stabilized with *β-*lactoglobulin amyloid fibrils in colloidal state.
   It is considered particularly advantageous using such green chemistry for manufacturing elemental gold.

In a **third aspect,** various uses of the composite materials and to corresponding articles are described. As outlined above, the present invention provides a new class of multi-functional hybrid nanocomposite films made of 2D and 1D nanoscale building blocks: single crystal gold nanoplatelets and amyloid fibrils, making it useful in a very broad range of applications. Accordingly, films of the here-described composites can find applications in sensors, diagnostic devices, printed matter (printed paper), printed electronics, micromechanical devices and biological devices. This shall be explained in further detail below:
In one embodiment, the here-described composites may be used in **electric devices** as conducting materials. The amount of gold in the hybrid composite may be adjusted within virtually the entire composition range (e.g. from 1∼99 wt.%). Therefore, the hybrid composite is insulating when having a low gold content or metal conductive when having a high gold content. By changing the gold nanoplatelets content, the metal conductive window may be tuned within 10² ∼ 10⁵ S/cm.
In one further embodiment, the here-described composites may be used for **decoration, packaging and surface coating.** The hybrid composites of the present invention have the surfaces of homogenous metallic gold color, even at very low gold content of gold (e.g. 29 wt.%). All the composites produced according to this invention have smooth and shiny surfaces. This is attributed to the super-large size and nanoscale thickness of gold platelets as well as their super flat surfaces.
In one further embodiment, the here-described composites may be used as **actuator to control air humidity.** Due to the presence of amyloid fibrils in the hybrid composites, water can diffuse easily within. For example, the composite with 87 wt. % gold has a dependence of the conductivity on the relative humidity. The resistance increased in up to 2 orders of magnitude with the relative humidity over 85 %. By micro-scratching the film with 90 wt. % gold, upon cyclic changes in humidity, the hybrid film may exhibit "ON" and "OFF" conductivity states. Upon exposure to water, the scratched film swells, anneals and shows a resistance close to that of the unscratched film. Accordingly, the inventive composites may be used for sensors responsive to water.
Gold platelets in the hybrid composites may also have the change of surface plasmon resonance upon exposure to water and the pH changes. For example, due to the lower reflective index of water compared with protein, the resonance peak of gold platelets undergoes a blue shift and becomes more pronounced upon exposure of the film (29 wt. % gold) to water. This resonance peak also varies by changing the pH of the rinsing solution, due to the existence of the isoelectric point of β-lactoglobulin (pH 5.1) as well as stronger optical coupling between gold platelets. Accordingly, the inventive composites may be used for **sensors** responsive to pH change.
In one further embodiment, the here-described composites may be used as an **ink.** In this embodiment, the composites are formulated as a suspension. Due to water processing method, the stable colloidal suspension can be used as ink for printing gold on a substrate.

The here-described hybrid composites may also be fluorescent.

In a further embodiment, an article is described, comprising a substrate and a coating, said coating consisting of an inventive composite material as described herein.
Such articles include decorative articles, such as packaging materials, decorative articles which are partly or fully coated with the inventive composite or which are printed with an ink comprising the inventive composite.

## Claims

1. A method for manufacturing a composite material
said composite material comprising amyloid fibrils and elemental gold, wherein
(i) said composite material is in the form of a self-supporting thin film or in the form of a coating on a substrate; and
(ii) said elemental gold is present as single crystal gold platelets; and
said method comprising the steps of:
(a) growing protein amyloid fibrils by incubating *β-*lactoglobulin solution for 6 h under pH ∼ 2, low ionic strength (I ≤ 20 mM), high temperature (T ∼ 90 °C) and the protein concentration (2 wt.%); and
(b) growing single crystal platelets by mixing 0.67 wt. % amyloid fibrils and 0.066 wt.% chloroauric acid, and then incubating at pH 2 and temperature 60 °C for 12 h; and
(c) optionally concentrating single crystal gold platelets by centrifugation and removing part of upper supernatant amyloid fibrils solution; and then either
(d) filtrating the gold platelets-protein fibrils suspension through filtration membranes and drying naturally in air; or
(e) printing the gold platelets-protein fibrils suspension into a patterned solid film motif on a substrate; or
(f) casting the gold platelets-protein fibrils suspension.

2. The method according to claim 1, **characterized in that** said composite material is in the form of a coating on a substrate.

3. The method according to claim 1 or 2, wherein said amyloid fibrils
(a) have high aspect ratio, with ≤ 10 nm in diameter and ≥ 1µm in length, and / or
(b) have highly charged surfaces, with electrophoretic mobilities of the order of 2 µm·cm/V·s at pH 4.

4. The method according to any of the preceding claims, wherein said gold
(a) is present as single crystal gold platelets, with the size up to 20 µm and the thickness only of 100 nm or less; and/or
(b) amounts to 10-99 weight-% of the total weight of the composite material.

5. The method according to any of the preceding claims, wherein
(a) said elemental gold is present in single crystal platelets and
(b) gold platelets and amyloid fibrils are well-organized into layered packing structures in films.

6. The method according to claim 1, wherein the composite material is obtained in the form of
(a) a self-supporting thin film;
(b) a coating on a substrate;
(c) a patterned coating motif on a substrate.

## Patentansprüche

1. Ein Verfahren zur Herstellung eines Verbundmaterials, wobei
das Verbundmaterial Amyloidfibrillen und elementares Gold umfasst, wobei
(i) das Verbundmaterial in der Form einer selbsttragenden dünnen Folie oder in der Form einer Beschichtung auf einem Substrat ist; und
(ii) das elementare Gold als einzelne Kristallgoldplättchen vorhanden ist; und
wobei das Verfahren die Schritte:
(a) Züchten von Proteinamyloidfibrillen durch Inkubation einer β-Lactoglobulin-Lösung für 6 h bei pH ∼ 2, niedriger Ionenstärke (I ≤ 20 mH), hoher Temperatur (T ∼ 90 °C) und der Proteinkonzentration (2 Gew.%); und
(b) Züchten von einzelnen Kristallplättchen durch Mischen von 0.67 Gew.% Amyloidfibrillen und 0.066 Gew.% Tertachlorogoldsäure, und anschliessende Inkubation bei pH 2 und einer Temperatur von 60 °C für 12 h; und
(c) optional Anreichern von einzelnen Kristallgoldplättchen durch Zentrifugieren und Entfernen eines Teils der oberen überstehenden Amyloidfibrillenlösung; und
anschliessend entweder
(d) Filtrieren der Goldplättchen-Proteinfibrillen-Suspension durch Filtrierungsmembrane und natürliches Trocknen an der Luft; oder
(e) Drucken der Goldplättchen-Proteinfibrillen-Suspension in ein strukturiertes, festes Folienmotiv auf einem Substrat; oder
(f) Giessen der Goldplättchen-Proteinfibrillen-Suspension,
umfasst.

2. Das Verfahren nach Anspruch 1, **dadurch gekennzeichnet, dass** das Verbundmaterial in der Form einer Beschichtung auf einem Substrat ist.

3. Das Verfahren nach Anspruch 1 oder 2, wobei die Amyloidfibrillen
(a) ein hohes Aspektverhältnis mit ≤ 10 nm Durchmesser und ≥ 1 µm Länge haben, und/oder
(b) eine hochgradig geladene Oberfläche mit elektrophoretischen Beweglichkeiten in der Grössenordnung von 2 µm•cm/V•s bei pH 4 haben.

4. Das Verfahren nach einem der vorangehenden Ansprüche, wobei das Gold
(a) als einzelne Kristallgoldplättchen mit einer Grösse bis zu 20 µm und einer Dicke von nur 100 nm oder weniger vorhanden ist, und/oder
(b) 10-99 Gew.% des Gesamtgewichts des Verbundmaterials ausmacht.

5. Das Verfahren nach einem der vorangehenden Ansprüche, wobei
(a) das elementare Gold in einzelnen Kristallplättchen vorhanden ist und
(b) Goldplättchen und Amyloidfibrillen gut in geschichteten Packungsstrukturen in Folien organisiert sind.

6. Das Verfahren nach Anspruch 1, wobei das Verbundmaterial in der Form von
(a) einer selbsttragenden dünnen Folie;
(b) einer Beschichtung auf einem Substrat;
(c) eines strukturierten Beschichtungsmotiv auf einem Substrat
gewonnen wird.

## Revendications

1. Un procédé pour la fabrication d'un matériau composite,
ledit matériau composite comprenant des fibrilles amyloïdes et de l'or élémentaire,
(i) ledit matériau composite étant sous forme d'une pellicule mince autoportante ou sous forme d'un revêtement sur un substrat; et
(ii) ledit or élémentaire étant présent sous forme de plaquettes individuelles d'or cristallin; et
ledit procédé comprenant les étapes:
(a) de croître des fibrilles amyloïdes de protéine en incubant une solution de β-lactoglobuline pendant 6 h à pH ∼ 2, basse puissance ionique (I ≤ 20 mH), haute température (T ∼ 90 °C) et la concentration de protéine (2 % par poids); et
(b) de croître des plaquettes individuelles de cristal en mélangeant 0.67 % par poids des fibrilles amyloïdes et 0.066 % par poids d'acide tétrachloroaurique, et ensuite incubation à pH 2 et une température de 60 °C pendant 12 h; et
(c) optionnellement de concentrer des plaquettes individuelles d'or cristallin par centrifugation et enlèvement d'une partie supérieure surnageante de la solution de fibrilles amyloïdes; et ensuite soit
(d) de filtrer la suspension des fibrilles de protéine et plaquettes d'or à travers membranes de filtration et de sécher de manière naturelle à l'air; soit
(e) d'imprimer la suspension des fibrilles de protéine et plaquettes d'or dans un motif de pellicule solide structuré sur un substrat; soit
(f) de couler la suspension des fibrilles de protéine et plaquettes d'or.

2. Le procédé selon la revendication 1, **caractérisé en ce que** ledit matériau composite est sous forme d'un revêtement sur un substrat.

3. Le procédé selon la revendication 1 ou 2, lesdites fibrilles amyloïdes
(a) ayant un rapport d'aspect élevé, avec un diamètre ≤ 10 nm et une longueur ≥ 1 µm, et/ou
(b) ayant des surfaces très chargées, avec des mobilités électrophorétiques dans l'ordre de 2 µm•cm/V•s à pH 4.

4. Le procédé selon l'une des revendications précédentes, l'or
(a) étant présent sous forme de plaquettes individuelles d'or cristallin, avec une dimension jusqu'à 20 µm et l'épaisseur seulement de 100 nm ou moins; et/ou
(b) ayant un poids compris entre 10-99 % par poids du poids total du matériau composite.

5. Le procédé selon l'une des revendications précédentes,
(a) ledit or élémentaire étant présent dans des plaquettes de cristal individuelles et
(b) des plaquettes de cristal et des fibrilles amyloïdes étant bien organisées en pellicules dans des structures de conditionnement stratifiées.

6. Le procédé selon la revendication 1, le matériau composite étant obtenu sous forme
(a) d'une pellicule mince autoportante;
(b) d'un revêtement sur un substrat;
(c) d'un motif de revêtement structuré sur un substrat.
